# EUROPEAN PATENT SPECIFICATION

(11) **EP 1 894 557 B1**
(45) Date of publication and mention of the grant of the patent: **16.02.2011**
(21) Application number: 06018423.1
(22) Date of filing: 04.09.2006
(51) Int. Cl.: A61K 9/00, A61K 9/08, A61K 9/10, A61K 31/663, A61K 47/36, A61K 47/32, A61K 47/38

(54) **Liquid composition for prevention and/or treatment of different bone metabolic diseases, uses thereof, and preparation process therefore**
Flüssige Zusammensetzung für die Behandlung und/oder Verhinderung von verschiedenen metabolischen Knochenkrankheiten, Verwendung davon und Vorbereitungsprozess.
Composition liquide pour le traitement et/ou Prévention de différent maladies du métabolisme d'os, utilisation et procédé de préparation.

(43) Date of publication of application: 05.03.2008
(73) Proprietor: GADOR S.A., Buenos Aires 1414 (AR)
(72) Inventor: Diaz, Liliana Elizabeth, 1414 Buenos Aires (AR)
(74) Representative: Winkler, Andreas Fritz Ernst

(56) References cited:
- WO-A-02/058708
- US-A- 5 462 932

## Description

This invention relates to a liquid composition for the prevention and/or treatment of different bone metabolic diseases, such as metabolic osteopathies, uses thereof and a preparation process therefore.

Bisphosphonates are well known chemical compounds, used in medicine to prevent and treat different bone metabolic diseases, for instance metabolic osteopathies such as osteoporosis and cancerous metastasis, as well as those osteopathies associated with rheumatoid arthritis.

Bisphosphonates may exist in form of acids, salts, hydrates and aminoderivatives. Within the group of bisphosphonates we can mention monosodium alendronate, disodium/amidronate, monosodium olpadronate, neridronate, etidronate, clodronate, ibandronate, incadronate, rise-dronate, zoledronate, tiludronate, amino derivatives such as aminoalendronate, aminodimethylalendronate, aminopamidronate, aminoolpadronate, aminoetidronate, and similar ones. As regards the different medical indications of the bisphosphonates, we can highlight their use in preventing and treating the established osteoporosis (see Papapoulos, S. E., et al. The use of bisphosphonates in the treatment of osteoporosis; Bone 1992, 13: A41 - A49).

The medical uses imply the continuous administration or by pulses of the bisphosphonates, particularly of the alendronate during long periods of 5 years or more. The use of preparations which are appropriate for their oral administration are thus preferred. The continuous use or by pulses is understood as one boosting the inhibition of the bone metabolism in a uniform and constant way, as it has been demonstrated by the curve of stable levels of the bone metabolism's markers.

On the other hand, the bisphosphonates may be used in a discontinuous or cyclic way, which stimulates a larger depression of the bone metabolism, followed by a trend to recover from the basal level. With this manner, the curve of the biochemical markers fluctuates, presenting valleys and peaks delimited by each administration cycle.

In the continuous way, each administration of bisphosphonates can be marked as a pulse (see Roldan, E. J. A., et al. Clinical evaluation of bisphosphonates - pharmacokinetics -principles; Medicine (Buenos Aires) 1997, 57 (suppl.): 76 - 82.

While the pulses administrated once a day, once a week day, once a week or twice a week stimulate in the bone a continuous metabolic alteration, the monthly, bimonthly or quarterly ones boos a discontinuous or cyclic metabolic alteration. Although no exact definition of the limit between the cyclic and continuous manners is known, this invention's object is to improve the administration of the bisphosphonates by pulses.

The concept of administration of the bisphosphonates by pulses has also been a common clinical practice in patients with a reduced body mass' in children and in those not tolerating the daily administration (see Roldan, E. J. A., and col.: J Pediat. Endocrinol Metab. 1999, 12: 555-559).

Nevertheless, up date, the requirement of high doses of bisphosphonates has hampered the successful oral administration, particularly due to potential problems of digestive tolerance. On the other hand, most of the bisphosphonates have a very low solubility, 3% w/v or less. This favors a very poor absorption of the compound, which in the best of the cases means a 1% bioavailability. Consequently, 99% of the remaining active substance may easily form lumps and precipitate in the digestive light, which may cause digestive irritability. The symptoms resulting of this irritability may vary from unspecific problems until pain, vomiting and also esophagitis and gastritis.

The injuries of the digestive system may be related to the own potential of the bisphosphonate of producing irritation of the digestive mucose, with the solubility process or the dissolution of the pharmaceutical forms and with the amount of molecules administered each time (see Spivacow, R. S., et al. Tolerability of oral bisphosphonates in patients with osteosporosis and other osteopathies; Medicine (Buenos Aires) 1997, 57 (supl.): 114 - 118).

Attempts were made to improve the tolerance by prescribing low doses of bisphosphonates under very strict conditions, administering them at least 30 - 60 minutes before breakfast, with plenty of water. Besides, the patient must hold a strict fast and remain standing or sitting during all the time prior to breakfast. This rigid indications are certainly awkward and, together with the side effects, may lead to the lack of cooperation with the prescriptions. The patient only finds little motivation in continuing with an uncomfortable plan of administration which alters his usual life rhythm and which in most of the cases is addressed on preventing an uncertain fact, as is the probability of fractures due to osteoporosis at an old age. Short after starting the treatment the patient frequently abandons to observe the indications and fails, thus appearing side effects associated to the poor fulfillment of the prescription.

*Alendronate* is the 4-amino-1-hydroxybutylidene-1,1-bisphosphonic acid monosodium salt trihydrate and is an agent used to fight the bone reabsorption in bone diseases such as osteoporosis. This compound may be prepared according to that revealed in the patent's application AR000052A1 (Gador S.A.). The alendronate is a derivative of the bisphosphonates known since the 1970's; another method for its synthesis is presented for instance in US 4621077*,* where general formulations of capsules, effervescent granules and injectable solutions are furthermore described. In EP 402152 the alendronic acid's monosodium salt trihydrate is specifically revealed.

EP 336851 describes tablets of different kinds of bisphosphonates, with sodium lauryl sulfate as excipient, well known by expert technicians. The bioavailability of the forms of solid oral administration presents a disadvantage for some patients having problems to swallow these pharmaceutical forms. Besides, these patients comprise a significant percentage of the population of patients taking alendronate, bearing in mind that this drug is intended to a population of elderly women.

US 5462932 describes specific oral liquid formulations containing alendronate to facilitate the swallowing to people having difficulties to swallowing. The herein defined formulations are based on the presence of a complexing agent as is EDTA and/or the presence of citric acid as buffer or tampon. The special stabilizers were considered essential for the formulations of the bisphosphonates.

WO 02058708 (Gador S.A.) describes liquid compositions comprising at least one bisphosphonate, agents producing viscosity, at least one flavoring agent and purified water.

It is one of the objects of this invention to overcome the disadvantages of the old technique, particularly proportioning a stabilized liquid composition to prevent, treat and/or diagnose bone metabolic diseases, thus allowing a more comfortable administration of the bisphosphonate, in order to make the strict compliance with the prescriptions possible and to make the way to long-term clinical effects. On the other hand, another object of this invention is to reduce the risk of toxicity represented by the lumps caused by the degradation of the oral forms.

On the other hand, another object of this invention is to provide a process for the preparation of a liquid composition appropriate for the invention and to allow the use of such composition for the prevention, the treatment and/or the diagnosis of bone metabolic diseases.

It is an object of the present invention to overcome the drawbacks of the prior art, especially to provide a composition for prevention, treatment and/or diagnosis of metabolic diseases of bones which would enable a more comfortable administration of bisphosphonates, in order to make possible a strict fulfillment of the prescriptions and long-term clinical effects.

This object is achieved by a liquid composition according to claim 1. The use thereof is provided in claim 23 and the process for its preparation in claim 28. Preferred embodiments are disclosed in the sub-claims.

It was found that within the herein described group of bisphosphonates, the liquid compositions comprising the alendronate within the group of bisphosphonates, some agents producing viscosity and a flavoring agent and a coloring agent capable of providing a fruitlike appearance to the said composition, with a particularly orange flavoring, have a different behavior to the others. It is important in the liquid forms, that the composition presents such a viscosity being able to provide a form of supply with reproducibility in a container of easy use for the patients.

The viscosity and the adherence of the formulations of this invention is such that the said compositions present a great stability and resistance to washing. This invention's formulations are clear, stable, good tasting, and simple as well as cheap to prepare.

We have further found that the presence of the agent raising the viscosity in those liquid compositions of this invention allows obtaining more stable compositions and emphasizes the effect of the flavoring agent achieving a fruitlike appearance to the product. This allows to accept patients for the intake of such medicine, avoiding the problems which have been explained above.

Besides, the agent raising the viscosity contributes to provide a consistency allowing the patient to perceive a final formulation with more "body" compared to a simply aqueous solution. In technical terms this would mean an increase of the said liquid formulation's viscosity. This partly resembles in the product's appearance to the one of a natural juice, which as having pulp, is more "dense". Further to the consistency it contributes to the turbidity, and we can say that it contributes to stabilizing the system as, even if the essence contains elements insoluble in water, the same do not precipitate (nor do they separate from the system, for instance by flotation) at least in a detectable way.

Another advantage of the presence of the agents raising this composition's viscosity is to provide a large stability facing the pH and at the temperature, due latter one the viscosity doesn't alter (hence the product's appearance) in spite of the changes.

It is surprising that a chemically and physically stabilized composition has developed for the prevention and/or the treatment of bone metabolic diseases having an aspect, taste and consistency of a natural fruit juice in a simple and cheap manufacturing way. The composition does not need a control of the pH, as a determinant critical factor. The excipients included in the composition do not cause adverse interactions and maintain solubilized; in other words, they reduce to a minimum the risk of forming lumps or irritating particles and with the consecutive contact with the patients sensitive mucosa which, we indicate by the way, cannot remain upright. Due to its "nutritional" appearance, the composition is appropriately and sufficiently tasteful to be administered as a juice prior to breakfast, thus improving the treatment's compliance and helping to remember that the administration must occur before breakfast or some other meal, with which the collateral effects diminish and consequently the possibility of obtaining long-term therapeutic benefits increases. These two last concepts are based on the fact that in this invention all the included alendronate is dissolved in the composition.

According to this invention, the composition is potentially usable in children.

Otherwise, it is to be mentioned that this invention's composition does not require stabilizing agents, such as EDTA or citric acid. This invention's composition is stable in glass or polymer recipients. The fruitlike taste of this invention's composition can be obtained by means of a flavoring, such as Orange Meroar 74203, Lemon HS 24537, Raspberry 23306, Grapefruit AR-1536 or similar, and a selection of viscosity increasing agents alginate, propylglycolalginate, Arabic gum (acacia), xanthan gum, guar gum, locust beam, carrageenan gum, karaya gum, tragacanth gum, chitosan and carbomer or mixtures thereof. These synthetic excipients had not been used nor suggested before related to a composition with an active principle such as the alendronate or its pharmaceutically acceptable derivatives.

This invention refers to liquid stabilized pharmaceutical compositions for the prevention and/or the treatment of different bone metabolic diseases such as metabolic osteopathies.

In this invention the viscosity agents are non toxic anionic polymers, compatible with the remaining pharmaceutical ingredients of the composition, and have a very good stability and viscosity features throughout a long range of pH and temperature. They provide the final composition with an appropriate consistency. To achieve this technical effect they are used in concentrations of 0.02 to 0.12 w/v, being those preferred which have a value of 0.06% w/v. The amounts of the agents increasing the viscosity of this invention have been determined by means of empirical tests depending on the wanted features of the resulting composition. Specifically a sufficient amount must be used to achieve the effect of dispersion, suspension, solubility and stability of the compositions of this invention. Specific examples of the amounts of this agent in this invention can achieve very good results as the ones described in the examples 1 to 6.

**TABLE 1: Summary of the polymeric features of the agents producing viscosity of this invention**

| **Polymer Soluble in water** | **Structure** | **Ionic loading** | **PH Factor** | **Temperature Factor** | **Rheology** | **Relative Viscosity** | **Main Characteristics and differences** |
|---|---|---|---|---|---|---|---|
| Alginate, NF | Polysaccharide | anionic | Stable at pH 3-10 | Stable at low temperatures | Pseudo- plastic | Medium to high | Polyvalent Ions may affect it forming gels (crossed unions) |
| Propylgly-co-lalginate, FCC | Polysaccharide | anionic | Stable at pH 3-6, 7 | Stable at low temperatures | Pseudo-plastic | Medium to high | |
| Arabic gum (Acacia, NF) | Polysaccharide | anionic | Viscosity is affected by change of pH | Reversible loss of viscosity at high temperatures | Newto-nian at < 40% and pseudo-plastic at > 40% of conc. | Very low | Low viscosity; ability to occur at high concentration: excellent compatibility with sugars. |
| Carrageenan , FCC | Polysaccharide | anionic | Stable at pH 3-10 | Reversible loss of viscosity at High temperatures | Thixotro pic | Moderate | May get compound with milk casein; high possibility of gels formation |
| Guar gum, NF | Polysaccharide | anionic | Stable at pH 4-10 | Degradation of the viscosity at high temperature along time | Pseudo-plastic | High | High viscosity for natural gums |
| Karaya gum, FCC | Polysaccharide | anionic | Unsta-ble at pH < at 7 | Very low, the viscosity results irreversible at high temperatures | Thixotro pic | High | Insoluble, but swells in water; degradation to los pH's, limited life time |
| Locust bean gum | Polysaccharide | No iónico | | Degradation of the viscosity at high temperature along time | Pseudo-plastic | High | Subject to the de-polimerization, viscosity affected by the temperature |
| Tragacanth gum | Polysaccharide | anionic | Stable at pH 1.9- 8.5. Excellent Stability at low pH's | Good stability, loss of viscosity related to high temperature | Pseudo-plastic | High | Good enzymatic resistance, disaffectation of the viscosity due to changes of pH; high efficiency of viscosity at low concentrations. |
| Xanthan gum, NF | Polysaccharide | anionic | Good stability at pH 1-12 | Good stability, vistosity is not affected by changes of temperature | Pseudo-plastic | High | Good enzymatic resistance, disaffectation of the viscosity due to changes of pH; high efficiency of viscosity at low concentrations. |
| Chitosan | Acetylated quinine | anionic | Soluble only at pH < 6.0 | Good | Not reported | Moderate to low | Cationic; low tolerance to the anions, stable at low pH |
| Carbomer, USP | Poliacrilato reticulado | anionic | Soluble at pH > 4.0 | Good stability | Pseudo-plastic | Very high | Very efficient film former; low tolerance to the sales reduce rapidly the viscosity; good agent of suspension; organic solubility; excellent rheology for topical medicines |

Preferably, the viscosity agent is selected among xanthan gum, guar gum, carrageenan gum or mixtures thereof, and preferably xanthan gum. It must also be highlighted that those compositions are suitable when the amount of the viscosity agent lies between 0.02 to 0.12 % w/v, specially when it is available in an amount from 0.06 to 0.12 % w/v, and preferably 0.06 % w/v. The Xanthan gum is a polysaccharide with a high molecular weight produced through the fermentation of carbohydrates of pure cultures of microorganisms such as *xanthomonas camoestris.* This gum is available through a variety of trade forms, including Rhodigel (sold by Rhone Poulenc Industries) and Ketrol (sold by Kelco divison of de Merck & Co., Inc.) Monosodium alendronate trihydrate, is present in an amount of 0.05 % to 0.3 % w/v, and specially in an amount of 0.09135 % w/v, an amount of 0.268 % w/v and also an amount of 0.1218 % w/v.

Within this invention must be highlighted those stabilized liquid pharmaceutical compositions comprising a *flavoring agent* capable of providing a fruitlike taste to the composition of orange flavor and consisting in a liquid artificial essence, such as for instance Meroar 74203 orange peel essence. Typical flavoring agents which are commonly used in sweetened pharmaceuticals, foods, candies, beverages are also useful in the present invention; these materials may impart flavors such as lemon, raspberry, grapefruit and many others are within the scope of the present invention. The preferred flavoring agent are Lemon HS 24537, Raspberry 23306, Grapefruit AR-1536 or similar Those formulations are preferred where the amount of flavoring agent is comprised between 0.10 % and 0.50 % w/v, preferably over 0.30 % w/v, and specially an amount of 0.35 % w/v. This formulation may furthermore contain a yellow coloring such as FD&C yellow # 6 which synonymities are E110 or Sunset yellow. The composition's final appearance is thereby reinforced in order to make it look like the one of a natural fruit juice and for this purpose it is preferred to use smaller amounts to 0.03 % w/v, preferably 0.021 % w/v of the said yellow coloring, and very specially an amount of 0.006 % w/v. The preferred coloring agent is Sunset yellow of FCF, which molecular formula is C₁₆H₁₀N₂NA₂O₇S₂. Its molecular weight is 452.37 g and its CAS number is [2783-94-0]. It is also known as E110, FD&C yellow #6, 6-hydroxy-5-[(4-sulfophenyl)azo]-2-naphtalensulfonic acid as the disodium salt, disodium salt of the p-sulfophenylazo-2-naphtol-6-sulfonic acid, yellow orange S. Its physical features are: maximum absorption: 482 nm, color index number: CI 15985, purity (EC): matter insoluble in water: ≤0.2%, purity (US): sodium chloride and sulfate ≤5.0%, ether extracts ≤0.2%, mix of oxides ≤1.0%, subsidiary colorings ≤5.0%, total color: ≥85.0%, volatile material: ≤10.0% a 135°C, matter insoluble in water: ≤0.5%.

**Table 2: Solubility of the coloring agent**

| **Solvent** | **Solubility at 25°C** |
|---|---|
| Acetone | 1:38.5 |
| Ethanol (75%) | 1:333 |
| Glycerin | 1:5 |
| Propylenglycol | 1:45.5 |
| Propylenglycol (50%) | 1:5 |
| Water | 1:5.3 a 2°C |
| | 1:5.3 a 25°C |
| | 1:5 a 60°C |

Incompatibilities: low compatibility with citric acid, solutions of saccharose and saturated sodium bicarbonate solutions. Incompatibility with ascorbic acid, gelatine and glucose.

Preferred coloring agents are D&C yellow #10 (quinoline yellow), FD&C #40 (allura red), and FD&C #3 (erythrosine).

The composition of this invention may contain one or more *sweetening agents,* one or more *preserving agents,* and one or more *essence cosolvents,* among others. Another aspect of the invention is related to a composition for oral administration in liquid form which comprises the alendronate, the agent increasing the viscosity , the flavoring agent and the purified water, together with one or more preservatives, with one or more sweeteners, with one or more cosolvents. When the formulation of this invention contains such cosolvent, a certain class of preserving agents may be dissolved in this cosolvent, and this avoid to heat the water over 85°C (some preserving agents of the family of the parabens such as the methylparaben and the propylparaben must be dissolved on water at high temperatures which is a difficulty of the technique). Including such cosolvent has the advantage of avoiding this difficulty.

The *"sweetening agents"* may be cyclamate or its pharmaceutically acceptable salts, saccharine or its pharmaceutically acceptable salts, sucralose, aspartame, dextrose, maltose, fructose, galactose, among others. The following are preferred, for instance: sodium cyclamate and particularly when found in an amount of 0.10 a 0.20 % w/v, preferably 0.14 % w/v, sodium saccharin and it is pointed out when found in amount of 0.075 to 0.60 % w/v, preferably 0.14 % w/v, and sucralose and specially when found in an amount of 0.010 a 0.030 % w/v, pref erably 0.02 % w/v. The sodium cyclamate is a sweetening agent 30 times superior to the sucrose, being commonly used in the pharmaceutical formulations without known incompatibilities. The sodium saccharine is an intense sweetening agent and this salt is considered more soluble in water than the saccharine being the one most used in pharmaceutical formulations. The sweetening power is 300 times superior to the sucrose. The concentration range usually acceptable in oral solutions as the one of this invention is 0.075 - 0.6% w/v. Combined with cyclamate, lower values are nevertheless required to achieve the product's sweetening effect. The preferred concentration is 0.014% to minimize the disagreeable sensation and the bitter propriety of the same.

The *"preserving agents*" may be selected amont ethylparaben, methylparaben, propylparaben, sodium methylparaben, sodium propylparaben and sodium butylparaben. Particularly pointed out are the formulations when one of the preserving agents is the methylparaben and it is found in an amount of 0.03 to 0.1% w/v, and when another one of the preserving agents is the propylparaben and it is found in an amount of 0.01 to 0.02 % w/v. The parabens are antimicrobial preservatives effective over a wide range of pH and have a wide spectrum antimicrobial activity. The antimicrobial activity augments when the length of the alkylic chain increases, but nevertheless the aqueous solubility decreases. Mixes of parabens are generally used to achieve an effective preservation.

Other preservative agents which may be used include sodium benzoate and potassium sorbate. The sodium benzoate for instance is used in oral preparations in concentrations of 0,02 -0,5 %. Its effectivity is limited to an acidic pH (2 - 5), as its antimicrobial properties are associated to the non dissociated form (benzoic acid). Likewise, the potassium sorbate is used in concentrations of 0,1 - 0,2%, and to exert its action (as sorbic acid) the pH must be under 6. Both salts are more widely used than the corresponding acids due to its larger aqueous solubility. For the use of such preservative agents consequently the formulation may require the aggregate of an agent regulating the pH as for instance the citric acid, in appropriate amounts to achieve a pH of around 4 - 5. This acid may additionally favorable contribute to achieve a more natural citric fruit taste.

The *"cosolvent"* of the preservative agents may be ethanol or propylenglycol, preferably propylenglycol. That formulation comprising the said cosolvent in an amount of 1% is of particular interest.

Another aspect of this invention comprises the pharmaceutical composition characterized because it is a liquid suspension, particularly that liquid suspension containing the active principle dissolved by one or more excipients found disperse and conferring an opalescent appearance to the product, and which is free of coarse particles of suspension.

The *"sweetening agents"* may be cyclamate or its pharmaceutically acceptable salts, saccharine or its pharmaceutically acceptable salts, sucralose, aspartame, dextrose, maltose, fructose, galactose, among others. Preferred as those as for instance: sodium cyclamate and specially when found in an amount of 0.10 to 0.20 % w/v, preferably 0.14 % w/v, sodium saccharine and it is pointed out when found in an amount of 0.075 to 0.60 % w/v, preferably 0.014 % w/v, and sucralose and specially when found in an amount of 0.010 to 0.020 % w/v, preferably 0.02 % w/v. the sodium cyclamate is a sweetening agent 30 times superior to the sucrose, being commonly used in the pharmaceutical formulations without known incompatibilities. The sodium saccharine is an intense sweetening agent, and this salt is considered more soluble in water than the saccharine being the one most used in pharmaceutical formulations. The sweetening power is 300 times superior to the sucrose. The concentration range usually acceptable in oral solutions such as the of this invention is 0.075 - 0.6% w/v. In combination with cyclamate, there are nevertheless required lower values to achieve the product's sweetening effect. The preferred concentration is 0.014% to minimize the disagreeable sensation and the bitter propriety of the same. Another of the agents used is the sucralose, which may be added to the media to mask a disagreeable taste. To improve the stability and the median life of the compositions of this invention, it is suggested to use high purity sweetening agents. For those patients not tolerating high levels of sucralose, other sweetening agents mentioned in this request may be included, such as for instance saccharine or aspartame.

The *"preservative agents*" may be selected among the ethylparaben, methylparaben, propylparaben, sodium methylparaben, sodium propylparaben and sodium butylparaben. Particularly highlighted are the formulations when one of the preservative agents is methylparaben and it is found in an amount of 0.03 to 0.1 % w/v, and when another one of the preservative agents is propylparaben and is found in an amount of 0.01 to 0.02 % w/v. The parabens are effective antimicrobial preservatives over a wide range of pH and have a wide spectrum antimicrobial activity. The antimicrobial activity augments when the length of the alkylic chain increases, but nevertheless decreases the aqueous solubility. Mixes of parabens are generally used to achieve an effective preservation.

The *"cosolvent"* of the preservative agents may be ethanol, glycerol or propylenglycol, preferably propylenglycol. The formulation comprising the cosolvent in an amount of 1% is of particular interst.

Another aspect of this invention comprises the stabilized pharmaceutical composition because it is a liquid suspension, specially that liquid suspension containing the active alendronate by one or more excipients which are dispersed and confer the product an opalescent appearance, and is free of coarse particles of suspension..

Another embodiment comprises the stabilized liquid pharmaceutical compositions which may contain *at least one form of vitamin D.* The term "vitamin D" comprises not activated forms of vitamin D₂ (ergocalciferol), and vitamin D₃ (colecalciferol). By "not activated metabolites of the vitamins D₂ and/or D₃" are understood those hydroxylated forms of the vitamin D₂ (ergocalciferol), and vitamin D₃ (colecalciferol), also the derivatives 25-hydroxy-vitamin D₃ and 24,25-dihydroxy-vitamin D₃. The not activated metabolites are the forms of primary storage of the vitamin D₃ in the human body. The derivative 25-hydroxy-vitamin D₃ may be hydroxylated in the human body at the form 1,25-dihydroxy-vitamin D₃. The term "IU" means international units. One microgram of vitamin D is equivalent to approx. 40 IU. So 2800 IU of vitamin D₃ is equivalent to 70 µg vitamin D₃. Now, if the derivative 25-hydroxi-vitamin D₃ is approx. 1.4 times more powerful than the vitamin D₃, 50 µg of 25-hydroxy-vitamina D₃ can be equivalent to 70 µg vitamin D₃ (2800 IU).

For human beings, the compositions containing alendronate, its pharmaceutically acceptable salts, derivatives and its hydrates, and a non activated metabolite of vitamin D₂ and/or D₃, contain between 100 IU up to 60000 IU. Not limiting examples of these metabolites may include amounts of 1400 IU, 2800 IU, 4200 IU, 5600 IU, 7000 IU, 8400 IU, 14000 IU, and 28000 IU combined with different amounts of at least one bisphosphonate. These combinations may comprise therapeutically effective amounts of alendronate, its pharmaceutically acceptable salts, derivatives and its hydrates, with amounts superior to 20 mg and up to 200 mg, based on the pharmaceutically active alendronic acid.

As the different forms of vitamin D are unstable in an aqueous media, the liquid formulations containing both referred actives, comprise untimely preparations. One of the possible forms is the one where the alendronate is found in an oral liquid formulation as the one previously described and the vitamin D (alone or with pharmaceutically acceptable excipients favoring its dosage) is found in the cap, which contains a device which when pressure is applied to it, opens and the vitamin falls into the liquid, and which then must be appropriately dispersed by agitation. This operation is made immediately before the ingestion of the pharmaceutical composition. Preferred forms of this invention contain coated vitamin D which favors its physical-chemical stabilization. Another possibility is that the vitamin D is in a separate container dissolved in oily solvents. The solutions of alendronate and vitamin D would be immediately mixed prior to its intake. In this case the formulation may require the inclusion of appropriate emulsifiers and antioxidants well known by the expert in the technique.

The pharmaceutical composition of the invention may be prepared using common techniques and manufacturing processes generally known in the technique, although those comprising the following steps are particularly preferred:
a. Load the purified water into a reactor having an appropriate capacity. Start agitation and heating up to 85 - 90 °C.
b. Add the preservative agents and continue agitating until the dissolution has been completed.
c. Cool the product obtaining a temperature between 30 - 35°C.
d. Add the active principle: alendronate and continue agitating until the dissolution has been completed.
e. Add the viscosity increasing agent in form of rain under continuous agitation. Keep up the agitation until all the material has been dispersed.
f. Incorporate the sweetening agents and agitate until the dissolution has been completed.
g. In a separate recipient, dissolve the Yellow coloring in a fraction of purified water. Add to the recipient of the step f and add until achieving the homogenization of the solution.
h. Add the orange flavoring and agitate until achieving the homogenization. Control the temperature of the product obtained: this one must be under 30° C, if necessary it may be cooled down.
i. Add purified water until obtaining a theoretical volume of the lot and agitate until homogenizing. Measure the pH.
j. Fill into appropriate recipients up to a theoretical volume of 100 mL.

Besides, the liquid stabilized pharmaceutical composition of this invention is used for the prevention and/or the treatment of bone metabolic diseases. Preferred is also the use of the composition to prepare a medicine intended to the administration by pulses, which is intended to the use in metabolic osteopathies, which is intended to the use for osteoporosis, which is intended to the use in children, to the use as a beverage prior to the food intake.

Under "pharmaceutically acceptable salts" are understood those forms which are commonly used in the pharmaceutical compositions, such as for instance the alkaline metal salts (sodium, potassium), organic bases (such as N-methylglucamine) and aminoacids (as lisine).

Under "bone metabolic diseases" are understood those related to the bones restructuring or bone disorders, for instance, the Pager's disease, osteoporosis, metastatic bone diseases, malign hypercalcemia, periprosthetic osteolysis, periodontal disease, arthritic conditions, and others such as minimizing the potential irritation of the esophagus and further gastrointestinal adverse effects.

Under "to be free of coarse particles of suspension" is understood that no particle is evident through a visual examination of the formulation.

### EXAMPLES

### Example 1: Preparation process of a stabilized oral liquid composition of alendronate as medicinal fruit juice

The preparation process of the liquid pharmaceutical composition of this invention may include the following steps:
i. Load the purified water into a reactor having an appropriate capacity. Start agitation and heating up to 85 - 90 °C.
ii. Add to the reactor the preservative agents with agitation. Continue agitating until the dissolution has been completed.
iii. Cool the product up to a temperature between 30 - 35 °C. Add the active principle to the reactor, with agitation.
iv. Continue agitating until the dissolution has been completed. With ongoing agitation, add to the reactor the viscosity increasing agent in form of rain under continuous agitation.
v. In a recipient having an appropriate capacity, prepare a solution with purified water and the yellow coloring and add it to the reactor, with agitation. Later, also in a recipient having an appropriate capacity, prepare a solution with purified water and a first sweetening agent and add it to the reactor, with agitation.
vi. Under ongoing agitation, add to the reactor the second sweetening agent. Also, under continuous agitation, add to the reactor the flavoring agent. Finally, interrupt the agitation.
vii. Add to volume of 100 mL by aggregating purified water. Agitate to homogenize the obtained liquid suspension.

**Example 2:** A stabilized liquid composition of 70mg/100 mL according to this invention may include the following components and its respective quantities:

| | |
|---|---|
| Alendronate (as monosodium alendronate trihydrate) | 0.09135% w/v |
| Methylparaben | 0.080% w/v |
| Propylparaben | 0.020% w/v |
| Xanthan gum | 0.06% w/v |
| Orange Meroar 74203 | 0.35% w/v |
| Sucralose | 0.020% w/v |
| Sodium cyclamate | 0.14% w/v |
| FD&C yellow #6 | 0.006% w/v |
| Purified water | sufficient amount |

**Example 3:** A stabilized liquid composition of 70mg/75 mL according to this invention may include the following components and its respective quantities:

| | |
|---|---|
| Alendronate (as monosodium alendronate trihydrate) | 0.1218% w/v |
| Sodium benzoate | 0.10% w/v |
| Xanthan gum | 0.06% w/v |
| Lemon HS 24537 (Fritzsche) | 0.35% w/v |
| Sodium saccharin | 0.025% w/v |
| Sodium cyclamate | 0.10% w/v |
| D&C yellow #10 | 0.005% w/v |
| Purified water | sufficient amount |

**Example 4:** A stabilized liquid composition according to this invention may include the following components and its respective quantities:

| | |
|---|---|
| Alendronate (as monosodium alendronate trihydrate) | 0.0268% w/v |
| Methylparaben | 0.080% w/v |
| Propylparaben | 0.020% w/v |
| Xanthan gum | 0.06% w/v |
| Raspberry 23306 (Fritzsche) | 0.35% w/v |
| Sucralose | 0.030% w/v |
| FD & C #40 | 0.004% w/v |
| Purified water | sufficient amount. |

**Example 5:** A stabilized liquid composition of 70mg/100 mL according to this invention may include the following components and its respective quantities:

| | |
|---|---|
| Alendronate (as monosodium alendronate trihydrate) | 0.09135% w/v |
| Methylparaben | 0.080% w/v |
| Propylparaben | 0.020% w/v |
| Xanthan gum | 0.12% w/v |
| Grapefruit AR-1536 (Robertet) | 0.35% w/v |
| Sodium saccharin | 0.020% w/v |
| Sodium cyclamate | 0.14% w/v |
| D&C #10 | 0.006% w/v w/v |
| Purified water | sufficient amount |

**Example 6:** A stabilized liquid composition of 70 mg/75 mL according to this invention may include the following components and its respective quantities:

| | |
|---|---|
| Alendronate (as monosodium alendronate trihydrate) | 0.1218% w/v |
| Methylparaben | 0.080% w/v |
| Propylparaben | 0.020% w/v |
| Xanthan gum | 0.12% w/v |
| Orange Meroar 74203 | 0.35% w/v |
| Sodium saccharin | 0.020% w/v |
| Sodium cyclamate | 0.14% w/v |
| FD&C yellow #°6 | 0.006% w/v |
| Purified water | sufficient amount |

### Example 7: Stability tests according to example 2

The following stability tests have been carried out for the stabilized liquid compositions of 70 mg/100 mL of this invention which characteristics are: container: colorless glass flask type III, flask's volume: 125 mL, security device: white polypropylen cap with sheathing of the container: foamed polyethylene with double film
A. Storage conditions in prolonged period at 25°C and 60% relative humidity

| **Characteristics** | **Specifications** | **Time (months)- Results** | | | | |
|---|---|---|---|---|---|---|
| | | **0** | **3** | **6** | **9** | **12** |
| Appearance of the solution (color, odor, taste) | Compared with standard | Conforms | Conforms | Conforms | Conforms | Conforms |
| Resuspendibility | No caking or phase separation | Conforms | Conforms | Conforms | Conforms | Conforms |
| PH | 14.0-7.0 | 5.0 | 5.5 | 5.0 | 5.1 | 5.3 |
| Chromatographic impurities | Phosphate NMT 1.0% | <0.05 | <0.05 | 0.1 | 0.2 | 0.1 |
| | Unknown NMT 0.2% | <0.05 | <0.05 | <0.05 | <0.05 | <0.05 |
| | Impurities total NMT 1.0% | <0.05 | <0.05 | 0.1 | 0.2 | 0.1 |
| Methylparaben | 0.72-0.88 mg/mL | 0.79 | 0.78 | 0.79 | 0.79 | 0.82 |
| Propylparaben | 0.18-0.22 mg/mL | 0.20 | 0.19 | 0.20 | 0.19 | 0.20 |
| Alendronate assay | 90.0-110% | 102.0 | 100.2 | 100.3 | 99.5 | 101.0 |
| Microbial limit test | Aerobics total count NMT 100 CFU/mL | <100 | | | | <100 |
| | Molds and yeasts NMT 100 CFU/mL | <100 | | | | <100 |
| | Enterobactereaceae NMT 100 CFU/mL | Conforms | | | | Conforms |
| | Pathogenic: absent in 1¹ | Conforms | | | | Conforms |

| | | | | | | |
|---|---|---|---|---|---|---|
| ¹ Pathogenics: E. Coli, Salmonella sp., Paeruginose,S. aureus | | | | | | |

B. Intermediate conditions at 30°C and 75% relative humidity

| **Characterics** | **Specifications** | **Time (months)- Results** | | | | |
|---|---|---|---|---|---|---|
| | | **0** | **3** | **6** | **9** | **12** |
| Appearance of the solution (color, odor, taste) | Compared with standard | Conforms | Conforms | Conforms | Conforms | Conforms |
| Resuspendibility | No caking or phase separation | Conforms | Conforms | Conforms | Conforms | Conforms |
| pH | 4.0-7.0 | 5.0 | 5.5 | 5.4 | 5.3 | 5.3 |
| Chromatographic impurities | Phosphate NMT 1.0% | <0.05 | <0.05 | <0.05 | <0.05 | 0.1 |
| | Unknown NMT 0.2% | <0.05 | <0.05 | <0.05 | <0.05 | <0.05 |
| | Impurities total NMT 1.0% | <0.05 | <0.05 | <0.05 | <0.05 | 0.1 |
| Methylparaben | 0.72-0.88 mg/mL | 0.79 | 0.78 | 0.78 | 0.80 | 0.80 |
| Propylparaben | 0.18-0.22 mg/mL | 0.20 | 0.19 | 0.20 | 0.19 | 0.20 |
| Alendronate test | 90.0-110% | 102.0 | 99.9 | 99.7 | 97.8 | 99.9 |
| Microbial limit test | Aerobics total count NMT 100 CFU/mL | <100 | | | | <100 |
| | Molds and yeasts NMT 100 cfu/mL | <100 | | | | <100 |
| | Enterobactereaceae NMT 100 CFU/mL | Conforms | | | | Conforms |
| | Pathogenic: absent in 1² | Conforms | | | | Conforms |

| | | | | | | |
|---|---|---|---|---|---|---|
| ² Pathogenics: E. Coli, Salmonella sp., Paeruginose,S. aureus | | | | | | |

C. Accelerated conditions at 40°C and 75% relative humidity

| **Characteristics** | **Specifications** | **Time (months)- Results** | | | | |
|---|---|---|---|---|---|---|
| | | **0** | **3** | **6** | **9** | **12** |
| Appearance of the solution (color, odor, taste) | Compared with standard | Conforms | Conforms | Conforms | Conforms | Conforms |
| Resuspendibility | No caking or phase separation | Conforms | Conforms | Conforms | Conforms | Conforms |
| pH | 14.0-7.0 | 5.0 | 5.2 | 5.6 | 5.6 | 5.5 |
| Chromatographic impurities | Phosphate NMT 1.0% | <0.05 | <0.05 | <0.05 | <0.05 | 0.1 |
| | Unknown NMT 0.2% | <0.05 | <0.05 | <0.05 | <0.05 | <0.05 |
| | Impurities total NMT 1.0% | <0.05 | <0.05 | <0.05 | <0.05 | 0.1 |
| Methylparaben | 0.72-0.88 mg/mL | 0.79 | 0.77 | 0.78 | 0.77 | 0.78 |
| Propylparaben | 0.18-0.22 mg/mL | 0.20 | 0.20 | 0.19 | 0.19 | 0.20 |
| Alendronate assay | 190.0-110% | 102.0 | 102.4 | 99.6 | 99.4 | 99.2 |
| Microbial limit test | Aerobics total count NMT 100 CFU/mL | <100 | | | | |
| | Molds and yeasts NMT 100 CFU/mL | <100 | | | | |
| | Enterobactereaceae NMT 100 CFU/mL | Conforms | | | | |
| | Pathogenic: absent in 1³ | Conforms | | | | |

| | | | | | | |
|---|---|---|---|---|---|---|
| Analytical process for impurities optimized at 6 months ³ Pathogenics: E. Coli, Salmonella sp., Paeruginose,S. aureus | | | | | | |

### Example 8: Stability tests according to example 5

The following stability tests have been carried out for the stabilized liquid compositions of 70 mg/100 mL of this invention which Characteristics are: colorless PET bottle container (terephtalate polyethylene, bottle size: 100 mL, security device: White polypropylene cap with foamed polyethylene sheathing with doble film
A. Storage conditions in prolonged period at 25°C and 60% relative humidity

| **Characteristics** | **Specifications** | **Time (months)- Results** | | | | |
|---|---|---|---|---|---|---|
| | | **0** | **3** | **6** | **9** | **12** |
| Appearance of the solution (color, odor, taste) | Compared with standard | Conforms | Conforms | Conforms | Conforms | Conforms |
| Resuspendibility | No caking or phase separation | Conforms | Conforms | Conforms | Conforms | Conforms |
| pH | 4.0-7.0 | 5.1 | 4.7 | 4.5 | 4.4 | 4.5 |
| Chromatographic impurities | Phosphate NMT 1.0% | <0.05 | <0.05 | 0.05 | 0.1 | 0.1 |
| | Unknown NMT 0.2% | <0.05 | <0.05 | <0.05 | <0.05 | <0.05 |
| | Impurities total NMT 1.0% | <0.05 | <0.05 | 0.05 | 0.05 | 0.1 |
| Methylparaben | 0.72-0.88 mg/mL | 0.78 | 0.78 | 0.78 | 0.81 | 0.79 |
| Propylparaben | 0.18-0.22 mg/mL | 0.19 | 0.19 | 0.19 | 0.19 | 0.19 |
| Alendronate assay | 90.0-110% | 98.5 | 98.1 | 99.1 | 100.9 | 101.6 |
| Microbial limit test | Aerobics total count not> 100 CFU/mL | <100 | | | | <100 |
| | Molds and yeasts not> 100 CFU/mL | <100 | | | | <100 |
| | Enterobactereaceae not> 100 CFU/mL | Conforms | | | | Conforms |
| | Pathogenic: absent in 1⁴ | Conforms | | | | Conforms |

| | | | | | | |
|---|---|---|---|---|---|---|
| ⁴ Pathogenics: E. Coli, Salmonella sp., Paeruginose,S. aureus | | | | | | |

B. Intermediate conditions at 30°C and 75% relative humidity

| **Characteristics** | **Specifications** | **Time (months)- Results** | | | | |
|---|---|---|---|---|---|---|
| | | **0** | **3** | **6** | **9** | **12** |
| Appearance of the solution (color, odor, taste) | Compared with standard | Conforms | Conforms | Conforms | Conforms | Conforms |
| Resuspendibility | No caking or phase separation | Conforms | Conforms | Conforms | Conforms | Conforms |
| PH | 14.0-7.0 | 5.1 | 4.7 | 4.5 | 4.7 | 4.4 |
| Chromatographic impurities | Phosphate NMT 1.0% | <0.05 | <0.05 | <0.05 | 0.2 | 0.1 |
| | Unknown NMT 0.2% | <0.05 | <0.05 | <0.05 | <0.05 | <0.05 |
| | Impurities total NMT 1.0% | <0.05 | <0.05 | <0.05 | 0.2 | 0.1 |
| Methylparaben | 0.72-0.88 mg/mL | 0.78 | 0.77 | 0.77 | 0.78 | 0.82 |
| Propylparaben | 0.18-0.22 mg/mL | 0.19 | 0.19 | 0.19 | 0.19 | 0.20 |
| Alendronate assay | 190.0-110% | 98.5 | 99.1 | 100.5 | 100.3 | 100.3 |
| Microbial limit test | Aerobics total count NMT 100 CFU/mL | <100 | | | | <100 |
| | Molds and yeasts NMT 100 CFU/mL | <100 | | | | <100 |
| | Enterobactereaceae NMT 100 CFU/mL | Conforms | | | | Conforms |
| | Pathogenic: absent in 1⁵ | Conforms | | | | Conforms |

| | | | | | | |
|---|---|---|---|---|---|---|
| ⁵ Pathogenics: E. Coli, Salmonella sp., Paeruginose,S. aureus | | | | | | |

C. Accelerated conditions at 40°C and 75% relative humidity

| **Characteristics** | **Specifications** | **Time (months)- Results** | | | | |
|---|---|---|---|---|---|---|
| | | **0** | **3** | **6** | **9** | **12** |
| Resuspendibility | No caking or phase separation | Conforms | Conforms | Conforms | Conforms | Conforms |
| pH | 14.0-7.0 | 5.1 | 4.8 | 4.6 | 4.5 | 4.5 |
| Chromatographic impurities | Phosphate NMT 1.0% | <0.05 | <0.05 | <0.05 | <0.05 | <0.05 |
| | Unknown NMT 0.2% | <0.05 | <0.05 | <0.05 | <0.05 | <0.05 |
| | Impurities total NMT 1.0% | <0.05 | <0.05 | <0.05 | <0.05 | <0.05 |
| Methylparaben | 0.72-0.88 mg/mL | 0.78 | 0.77 | 0.76 | 0.76 | 0.78 |
| Propylparaben | 0.18-0.22 mg/mL | 0.19 | 0.19 | 0.18 | 0.18 | 0.19 |
| Alendronate assay | 90.0-110% | 98.5 | 98.7 | 98.3 | 98.2 | 100.9 |
| Microbial limit test | Aerobics total count NMT 100 CFU/mL | <100 | | | | |
| | Molds and yeasts NMT 100 CFU/mL | <100 | | | | |
| | Enterobactereaceae NMT 100 CFU/mL | Conforms | | | | |
| | Pathogenic: absent in 1⁶ absent in | Conforms | | | | |

| | | | | | | |
|---|---|---|---|---|---|---|
| ⁶ Pathogenics: E. Coli, Salmonella sp., Paeruginose,S. aureus | | | | | | |

### Example 9: Stability tests according to example 3

The following stability tests have been carried out for the stabilized liquid compositions of 70 mg/75 mL of this invention which Characteristics are: container: colorless glass flask type III, flask's volume: 125 mL, security device: white polypropylene cap with foamed polyethylene sheathing with doble film
A. Storage conditions in prolonged period at 25°C and 60% relative humidity

| **Characteristics** | **Specifications** | **Time (months)- Results** | | | | |
|---|---|---|---|---|---|---|
| | | **0** | **3** | **6** | **9** | **12** |
| Appearance of the solution (color, odor, taste) | Compared with standard | Conforms | Conforms | Conforms | Conforms | Conforms |
| Resuspendibility | No caking or phase separation | Conforms | Conforms | Conforms | Conforms | Conforms |
| pH | 14.0-7.0 | 5.4 | 5.4 | 5.4 | 5.3 | 5.1 |
| Chromatographic impurities | Phosphate NMT 1.0% | <0.05 | <0.05 | 0.2 | 0.2 | 0.1 |
| | Unknown NMT 0.2% | <0.05 | <0.05 | <0.05 | <0.05 | <0.05 |
| | Impurities total NMT 1.0% | <0.05 | <0.05 | 0.2 | 0.2 | 0.1 |
| Methylparaben | 0.72-0.88 mg/mL | 0.79 | 0.79 | 0.78 | 0.80 | 0.79 |
| Propylparaben | 0.18-0.22 mg/mL | 0.19 | 0.19 | 0.19 | 0.19 | 0.19 |
| Alendronate assay | 90.0-110% | 102.0 | 100.7 | 98.6 | 102.0 | 99.8 |
| Microbial limit test | Aerobics total count NMT 100 CFU/mL | <100 | | | | <100 |
| | Molds and yeasts NMT 100 CFU/mL | <100 | | | | <100 |
| | Enterobactereaceae NMT 100 CFU/mL | Conforms | | | | Conforms |
| | Pathogenic: absent in 1⁷ | Conforms | | | | Conforms |

| | | | | | | |
|---|---|---|---|---|---|---|
| ⁷ Pathogenics: E. Coli, Salmonella sp., Paeruginose,S. aureus | | | | | | |

B. Intermediate conditions at 30°C and 75% relative humidity

| **Characteristics** | **Specifications** | **Time (months)- Results** | | | | |
|---|---|---|---|---|---|---|
| | | **0** | **3** | **6** | **9** | **12** |
| Appearance of the solution (color, odor, taste) | Compared with standard | Conforms | Conforms | Conforms | Conforms | Conforms |
| Resuspendibility | No caking or phase separation | Conforms | Conforms | Conforms | Conforms | Conforms |
| pH | 4.0-7.0 | 5.4 | 5.4 | 5.4 | 5.1 | 5.2 |
| Chromatographic impurities | Phosphate NMT 1.0% | <0.05 | <0.05 | 0.2 | 0.1 | 0.1 |
| | Unknown NMT 0.2% | <0.05 | <0.05 | <0.05 | <0.05 | <0.05 |
| | Impurities total NMT 1.0% | <0.05 | <0.05 | 0.2 | 0.1 | 0.1 |
| Methylparaben | 0.72-0.88 mg/mL | 0.79 | 0.77 | 0.79 | 0.79 | 0.81 |
| Propylparaben | 0.18-0.22 mg/mL | 0.19 | 0.19 | 0.20 | 0.19 | 0.19 |
| Alendronate assay | 190.0-110% | 102.0 | 99.5 | 99.0 | 101.4 | 101.6 |
| Microbial limit test | Aerobics total count NMT 100 CFU/mL | <100 | | | | <100 |
| | Molds and yeasts NMT 100 CFU/mL | <100 | | | | <100 |
| | Enterobactereaceae NMT 100 CFU/mL | Conforms | | | | Conforms |
| | Pathogenic: absent in 1⁸ | Conforms | | | | Conforms |

| | | | | | | |
|---|---|---|---|---|---|---|
| ⁸ Pathogenics: E. Coli, Salmonella sp., Paeruginose,S. aureus | | | | | | |

C. Accelerated conditions at 40°C and 75% relative humidity

| **Characteristics** | **Specifications** | **Time (months)- Results** | | | | |
|---|---|---|---|---|---|---|
| | | **0** | **3** | **6** | **9** | **12** |
| Appearance of the solution (color, odor, taste) | Compared with standard | Conforms | Conforms | Conforms | Conforms | Conforms |
| Resuspendibility | No caking or phase separation | Conforms | Conforms | Conforms | Conforms | Conforms |
| pH | 14.0-7.0 | 5.4 | 5.3 | 5.5 | 5.4 | 5.4 |
| Chromatographic impurities | Phosphate NMT 1.0% | <0.05 | <0.05 | <0.05 | <0.05 | 0.2 |
| | Unknown NMT 0.2% | <0.05 | <0.05 | <0.05 | <0.05 | <0.05 |
| | Impurities total NMT 1.0% | <0.05 | <0.05 | <0.05 | <0.05 | 0.2 |
| Methylparaben | 0.72-0.88 mg/mL | 0.79 | 0.76 | 0.77 | 0.77 | 0.75 |
| Propylparaben | 0.18-0.22 mg/mL | 0.19 | 0.20 | 0.18 | 0.18 | 0.19 |
| Alendronate assay | 190.0-110% | 102.0 | 100.8 | 101.9 | 99.3 | 100.5 |
| Microbial limit test | Aerobics total count NMT 100 CFU/mL | <100 | | | | |
| | Molds and yeasts NMT 100 CFU/mL | <100 | | | | |
| | Enterobactereaceae NMT 100 CFU/mL | Conforms | | | | |
| | Pathogenic: absent in 1⁹ absent in | Conforms | | | | |

| | | | | | | |
|---|---|---|---|---|---|---|
| ⁹ Pathogenics: E. Coli, Salmonella sp., Paeruginose,S. aureus | | | | | | |

### Example 10: Stability tests according to example 6

The following stability tests have been carried out for the stabilized liquid compositions of 70 mg/75 mL of this invention which Characteristics are: colorless PET bottle container (terephtalate polyethylene, bottle size: 100 mL, security device: White polypropylene with foamed polyethylene sheathing with doble film

### A. Storage conditions in prolonged period at 25°C and 60% relative humidity

| **Characteristics** | **Specifications** | **Time (months)- Results** | | | | |
|---|---|---|---|---|---|---|
| | | **0** | **3** | **6** | **9** | **12** |
| Appearance of the solution (colour, odor, taste) | Compared with standard | Conforms | Conforms | Conforms | Conforms | Conforms |
| Resuspendibility | No caking or phase separation | Conforms | Conforms | Conforms | Conforms | Conforms |
| pH | 4.0-7.0 | 5.4 | 4.6 | 4.6 | 4.6 | 4.5 |
| Chromatographic impurities | Phosphate NMT 1.0% | <0.05 | <0.05 | 0.2 | 0.1 | 0.1 |
| | Unknown NMT 0.2% | <0.05 | <0.05 | <0.05 | <0.05 | <0.05 |
| | Impurities total NMT 1.0% | <0.05 | <0.05 | 0.2 | 0.1 | 0.1 |
| Methylparaben | 0.72-0.88 mg/mL | 0.80 | 0.78 | 0.79 | 0.80 | 0.80 |
| Propylparaben | 0.18-0.22 mg/mL | 0.19 | 0.19 | 0.19 | 0.19 | 0.20 |
| Alendronate assay | 90.0-110% | 102.0 | 100.5 | 99.3 | 98.5 | 101.1 |
| Microbial limit test | Aerobics total count NMT 100 CFU/mL | <100 | | | | <100 |
| | Molds and yeasts NMT 100 CFU/mL | <100 | | | | <100 |
| | Enterobactereaceae NMT 100 CFU/mL | Conforms | | | | Conforms |
| | Pathogenic: absent in 1¹⁰ | Conforms | | | | Conforms |

| | | | | | | |
|---|---|---|---|---|---|---|
| ¹⁰ Pathogenics: E. Coli, Salmonella sp., Paeruginose,S. aureus | | | | | | |

B. Intermediate conditions at 30°C and 75% relative humidity

| **Characteristics** | **Specifications** | **Time (months)- Results** | | | | |
|---|---|---|---|---|---|---|
| | | **0** | **3** | **6** | **9** | **12** |
| Appearance of the solution (color, odor, taste) | Compared with standard | Conforms | Conforms | Conforms | Conforms | Conforms |
| Resuspendibility | No caking or phase separation | Conforms | Conforms | Conforms | Conforms | Conforms |
| pH | 14.0-7.0 | 5.4 | 4.6 | 4.5 | 4.4 | 4.4 |
| Chromatographic impurities | Phosphate NMT 1.0% | <0.05 | <0.05 | 0.2 | 0.1 | 0.1 |
| | Unknown NMT 0.2% | <0.05 | <0.05 | <0.05 | <0.05 | <0.05 |
| | Impurities total NMT 1.0% | <0.05 | <0.05 | 0.2 | 0.1 | 0.1 |
| Methylparaben | 0.72-0.88 mg/mL | 0.80 | 0.78 | 0.78 | 0.76 | 0.80 |
| Propylparaben | 0.18-0.22 mg/mL | 0.19 | 0.19 | 0.19 | 0.19 | 0.19 |
| Alendronate assay | 90.0-110% | 102.0 | 101.6 | 99.3 | 101.4 | 100.9 |
| Microbial limit test | Aerobics total count NMT 100 CFU/mL | <100 | | | | <100 |
| | Molds and yeasts NMT 100 CFU/mL | <100 | | | | <100 |
| | Enterobactereaceae NMT 100 CFU/mL | Conforms | | | | Conforms |
| | Pathogenic: absent in 1¹¹ | Conforms | | | | Conforms |

| | | | | | | |
|---|---|---|---|---|---|---|
| ¹¹ Pathogenics: E. Coli, Salmonella sp., Paeruginose,S. aureus | | | | | | |

C. Accelerated conditions at 40°C and 75% relative humidity

| **Characteristics** | **Specifications** | **Time (months)- Results** | | | | |
|---|---|---|---|---|---|---|
| | | **0** | **3** | **6** | **9** | **12** |
| Appearance of the solution (color, odor, taste) | Compared with standard | Conforms | Conforms | Conforms | Conforms | Conforms |
| Resuspendibility | No caking or phase separation | Conforms | Conforms | Conforms | Conforms | Conforms |
| pH | 4.0-7.0 | 5.4 | 4.7 | 4.5 | 4.5 | 4.5 |
| Chromatographic impurities | Phosphate NMT 1.0% | <0.05 | <0.05 | <0.05 | <0.05 | 0.2 |
| | Unknown NMT 0.2% | <0.05 | <0.05 | <0.05 | <0.05 | <0.05 |
| | Impurities total NMT 1.0% | <0.05 | <0.05 | <0.05 | <0.05 | 0.2 |
| Methylparaben | 0.72-0.88 mg/mL | 0.80 | 0.76 | 0.77 | 0.77 | 0.79 |
| Propylparaben | 0.18-0.22 mg/mL | 0.19 | 0.20 | 0.19 | 0.19 | 0.19 |
| Alendronate assay | 90.0-110% | 102.0 | 101.1 | 100.7 | 102.1 | 100.7 |
| Limit microbial test | Aerobics total count NMT 100 CFU/mL | <100 | | | | |
| | Molds and yeasts NMT 100 CFU/mL | <100 | | | | |
| | Enterobactereaceae NMT 100 CFU/mL | Conforms | | | | |
| | Pathogenic: absent in 1¹² absent in | Conforms | | | | |

| | | | | | | |
|---|---|---|---|---|---|---|
| ¹² Pathogenics: E. Coli, Salmonella sp., Paeruginose,S. aureus | | | | | | |

According to the results set forth in the Examples 7 to 10 it may be observed that under the different conditions, no chemical and/or physical alterations to the composition object of this application have been observed.

On the other hand, it is possible to use another kind of food/beverage free of alcohol, as well as another flavoring liquid.

The prepared composition according to that described may be incorporated to a container of one unit or of several units or to a trade presentation highlighting that it is a medical use, so as to prevent it from being incorrectly used.

## Claims

1. Liquid composition for use in prevention and/or treatment of bone metabolic diseases, comprising:
alendronic acid or its acceptable pharmaceutical salts, or mixtures thereof, wherein the alendronic acid or its acceptable pharmaceutical salt is monosodium alendronate trihydrate, which is present in amount of 0.05% to 0.3% w/v,
at least one flavoring agent, purified water, and
a viscosity agent selected from the group consisting of alginate, propylglycolalginate, Arabic gum (acacia), xanthan gum, guar gum, locust bean, carrageenan gum, karaya gum, tragacanth gum, chitosan, sodium carboxymethyl cellulose and carbomer or mixtures thereof, **characterized in that** the viscosity agent is present in an amount of 0.02 to 0.12% w/v.

2. Liquid composition for use according to claim 1, wherein the viscosity agent is present in an amount of 0.06% w/v.

3. Liquid composition for use according to claim 1, wherein the monosodium alendronate trihydrate is present in amount of 0.09 to 0.27% w/v.

4. Liquid composition for use according to claim 1, wherein the flavoring agent is capable of providing the composition a fruit like flavor.

5. Liquid composition for use according to claim 4, wherein the flavoring agent is a flavoring orange liquid, lemon liquid, raspberry liquid, grapefruit liquid or red fruit liquid.

6. Liquid composition for use according to claim 1, further comprising a coloring agent.

7. Liquid composition for use according to claim 6, wherein the coloring agent is a yellow coloring agent.

8. Liquid composition for use according to claim 7, wherein the coloring agent is quinoline yellow.

9. Liquid composition for use according to claim 6 wherein the coloring agent is allura red or erythrosine.

10. Liquid composition for use according to claim 1, wherein the flavoring agent is present in an amount of 0.10% to 0.50% w/v, preferably 0.30 to 0.50% w/v.

11. Liquid composition for use according to claim 6, wherein the coloring agent is present in an amount of lower than 0.03% w/v, preferably in a range between 0.005% to 0.022% w/v.

12. Liquid composition for use according to claim 1, further comprising one or more sweetening agents, one or more preservative agents and/or one or more co-solvents.

13. Liquid composition for use according to claim 12, wherein the sweetening agent is selected from the group consisting of cyclamate or its pharmaceutically acceptable salts, saccharine or its pharmaceutically acceptable salts, sucralose, aspartame, dextrose, maltose, fructose, galactose, or mixtures thereof.

14. Liquid composition for use according to claim 13, wherein the sweetening agent is sodium cyclamate present in an amount of 0.10 to 0.20% w/v, preferably 0.14% w/v.

15. Liquid composition for use according to claim 13, wherein the sweetening agent is sodium saccharine present in an amount of 0.075 to 0.60% w/v, preferably 0.10 to 0.20% w/v.

16. Liquid composition for use according to claim 13, wherein the sweetening agent is sucralose present in an amount of 0.010 to 0.020% w/v, preferably 0.015 to 0.02% w/v.

17. Liquid composition for use according to claim 12, wherein the preservative agent is selected from the group consisting of ethyl parabene, methyl parabene, propyl parabene, sodium methyl parabene, sodium propyl parabene and sodium butyl parabene.

18. Liquid composition for use according to claim 17, wherein methyl parabene is present in an amount of 0.03 to 0.1% w/v and/or propyl parabene is present in an amount of 0.01 to 0.2% w/v.

19. Liquid composition for use according to claim 1, wherein the composition is a liquid suspension.

20. Liquid composition for use according to claim 1, wherein the co-solvent is present in an amount of 1% w/v, and is preferably propylene glycol.

21. Liquid composition for use according to claim 1, having a pH between 4.00 to 7.00.

22. Liquid composition for use according to claim 1, having a density of 0.980 to 1.02 g/ml at a temperature of 20°C.

23. Use of a composition according to any of the preceding claims for preparing a medicament for the prevention and/or treatment of bone metabolic diseases.

24. Use according to claim 23 for administration by pulses.

25. Use according to claim 23 or 24 for prevention and/or treatment of metabolic osteopathies or osteoporosis.

26. Use according to any of the claims 23 to 25 in children.

27. Use according to any of the claims 23 to 26 as a beverage prior to food intake.

28. Process for the preparation of liquid composition according to any of the claims 1 to 22, comprising the steps of:
(i) charging purified water into a reactor, adding preservative agent, and agitating until dissolution has been completed, and then optionally adjusting the temperature at between 30-35°C,
(ii) adding alendronate or its acceptable pharmaceutical salt and agitating until dissolution has been completed,
(iii) adding viscosity agent and agitating until all the material has been dispersed,
(iv) adding sweetening agent and agitating until dissolution has been completed,
(v) dissolving coloring agent in purified water in a separate recipient and adding to the mixture obtained in step (iv) until achieving homogenization of the mixture,
(vi) adding flavoring agent and agitating until homogenization is achieved, wherein the temperature after addition is below 30°C,
(vii) adding purified water until a theoretical volume of the lot is achieved and agitating until homogenization, and
(viii) filling into appropriate recipients up to a theoretical volume.

## Patentansprüche

1. Flüssige Zusammensetzung zur Verwendung bei der Verhinderung und/oder Behandlung von Knochenstoffwechselerkrankungen, umfassend:
Alendronsäure oder ihre verträglichen pharmazeutischen Salze, oder Mischungen davon, wobei die Alendronsäure oder ihr verträgliches pharmazeutisches Salz Mononatriumalendronat-Trihydrat ist, das in einer Menge von 0,05% bis 0,3% w/v vorliegt,
wenigstens einen Aromastoff, gereinigtes Wasser und
ein Viskositätsmittel, das ausgewählt ist aus der Gruppe, bestehend aus Alginat, Propylglykolalginat, Gummi arabicum (Akaziengummi), Xanthangummi, Guargummi, Johannesbrotgummi, Carrageenangummi, Karayagummi, Tragacanthgummi, Chitosan, Natriumcarboxymethylcellulose und Carbomer oder Mischungen davon, **dadurch gekennzeichnet, dass** das Viskositätsmittel in einer Menge von 0,02 bis 0,12% w/v vorliegt.

2. Flüssige Zusammensetzung zur Verwendung nach Anspruch 1, wobei das Viskositätsmittel in einer Menge von 0,06% w/v vorliegt.

3. Flüssige Zusammensetzung zur Verwendung nach Anspruch 1, wobei das Mononatriumalendronat-Trihydrat in einer Menge von 0,09 bis 0,27% w/v vorliegt.

4. Flüssige Zusammensetzung zur Verwendung nach Anspruch 1, wobei der Aromastoff in der Lage ist, der Zusammensetzung ein fruchtähnliches Aroma zu verleihen.

5. Flüssige Zusammensetzung zur Verwendung nach Anspruch 4, wobei der Aromastoff eine aromatisierende Orangenflüssigkeit, Zitronenflüssigkeit, Himbeerflüssigkeit, Grapefruitflüssigkeit oder Rotfruchtflüssigkeit ist.

6. Flüssige Zusammensetzung zur Verwendung nach Anspruch 1, die weiter ein Färbemittel umfasst.

7. Flüssige Zusammensetzung zur Verwendung nach Anspruch 6, wobei das Färbemittel ein gelbes Färbemittel ist.

8. Flüssige Zusammensetzung zur Verwendung nach Anspruch 7, wobei das Färbemittel Chinolingelb ist.

9. Flüssige Zusammensetzung zur Verwendung nach Anspruch 6, wobei das Färbemittel Allurarot oder Erythrosin ist.

10. Flüssige Zusammensetzung zur Verwendung nach Anspruch 1, wobei der Aromastoff in einer Menge von 0,10% bis 0,50% w/v, vorzugsweise 0,30 bis 0,50% w/v vorliegt.

11. Flüssige Zusammensetzung zur Verwendung nach Anspruch 6, wobei das Färbemittel in einer Menge von weniger als 0,03% w/v, vorzugsweise in einem Bereich zwischen 0,005% und 0,022% w/v vorliegt.

12. Flüssige Zusammensetzung zur Verwendung nach Anspruch 1, die weiter ein oder mehrere Süßungsmittel, ein oder mehrere Konservierungsmittel und/oder ein oder mehrere Cosolventien umfasst.

13. Flüssige Zusammensetzung zur Verwendung nach Anspruch 12, wobei das Süßungsmittel ausgewählt ist aus der Gruppe, bestehend aus Cyclamat oder seinen pharmazeutisch verträglichen Salzen, Saccharin oder seinen pharmazeutisch verträglichen Salzen, Sucralose, Aspartam, Dextrose, Maltose, Fructose, Galactose oder Mischungen davon.

14. Flüssige Zusammensetzung zur Verwendung nach Anspruch 13, wobei das Süßungsmittel Natriumcyclamat ist, das in einer Menge von 0,10 bis 0,20% w/v, vorzugsweise 0,14% w/v vorliegt.

15. Flüssige Zusammensetzung zur Verwendung nach Anspruch 13, wobei das Süßungsmittel Natriumsaccharin ist, das in einer Menge von 0,075 bis 0,60% w/v, vorzugsweise 0,10 bis 0,20% w/v vorliegt.

16. Flüssige Zusammensetzung zur Verwendung nach Anspruch 13, wobei das Süßungsmittel Sucralose ist, das in einer Menge von 0,010 bis 0,020% w/v, vorzugsweise 0,015 bis 0,02% w/v vorliegt.

17. Flüssige Zusammensetzung zur Verwendung nach Anspruch 12, wobei das Konservierungsmittel ausgewählt ist aus der Gruppe, bestehend aus Ethylparaben, Methylparaben, Propylparaben, Natriummethylparaben, Natriumpropylparaben und Natriumbutylparaben.

18. Flüssige Zusammensetzung zur Verwendung nach Anspruch 17, wobei Methylparaben in einer Menge von 0,03 bis 0,1% w/v vorliegt und/oder Propylparaben in einer Menge von 0,01 bis 0,2% w/v vorliegt.

19. Flüssige Zusammensetzung zur Verwendung nach Anspruch 1, wobei die Zusammensetzung eine flüssige Suspension ist.

20. Flüssige Zusammensetzung zur Verwendung nach Anspruch 1, wobei das Cosolvens in einer Menge von 1 % w/v vorliegt und vorzugsweise Propylenglykol ist.

21. Flüssige Zusammensetzung zur Verwendung nach Anspruch 1, die einen pH zwischen 4,00 bis 7,00 aufweist.

22. Flüssige Zusammensetzung zur Verwendung nach Anspruch 1, die eine Dichte von 0,980 bis 1,02 g/ml bei einer Temperatur von 20°C aufweist.

23. Verwendung einer Zusammensetzung nach einem der vorangehenden Ansprüche zur Herstellung eines Arzneimittels zur Verhinderung und/oder Behandlung von Knochenstoffwechselerkrankungen.

24. Verwendung nach Anspruch 23 zur Verabreichung in Pulsen.

25. Verwendung nach Anspruch 23 oder 24 zur Verhinderung und/oder Behandlung von Stoffwechselosteopathien oder Osteoporose.

26. Verwendung nach einem der Ansprüche 23 bis 25 bei Kindern.

27. Verwendung nach einem der Ansprüche 23 bis 26 als ein Getränk vor der Nahrungsaufnahme.

28. Verfahren zur Herstellung einer flüssigen Zusammensetzung nach einem der Ansprüche 1 bis 22, umfassend die Schritte:
(i) Einbringen von gereinigtem Wasser in einen Reaktor, Zugeben von Konservierungsmittel und Bewegen, bis die Auflösung abgeschlossen ist, und dann gegebenenfalls Einstellen der Temperatur auf zwischen 30-35°C,
(ii) Zugeben von Alendronat oder seinem verträglichen pharmazeutischen Salz und Bewegen, bis die Auflösung abgeschlossen ist,
(iii) Zugaben von Viskositätsmittel und Bewegen, bis alles Material dispergiert worden ist,
(iv) Zugeben von Süßungsmittel und Bewegen, bis die Auflösung abgeschlossen ist,
(v) Auflösen von Färbemittel in gereinigtem Wasser in einem getrennten Gefäß und Zugeben der Mischung, die in Schritt (iv) erhalten ist, bis Homogenisierung der Mischung erreicht ist,
(vi) Zugeben von Aromastoff und Bewegen, bis Homogenisierung erreicht ist, wobei die Temperatur nach der Zugabe unter 30°C liegt,
(vii) Zugeben von gereinigtem Wasser, bis ein theoretisches Volumen der Charge erreicht ist, und Bewegen bis zur Homogenisation und
(viii) Einfüllen in geeignete Gefäße bis zu einem theoretischen Volumen.

## Revendications

1. Composition liquide pour une utilisation pour la prévention et/ou le traitement de maladies métaboliques osseuses comprenant :
de l'acide alendronique ou ses sels pharmaceutiquement acceptables, ou des mélanges de ceux-ci, l'acide alendronique ou ses sels pharmaceutiquement acceptables étant l'alendronate monosodique trihydraté, qui est présent en une quantité de 0,05 % à 0,3 % m/v,
au moins un agent aromatisant, de l'eau purifiée, et
un agent de viscosité choisi dans le groupe constitué par un alginate, l'alginate de propylène glycol, la gomme arabique (acacia), la gomme xanthique, la gomme de guar, la caroube, la gomme de carraghénane, la gomme karaya, la gomme d'adragante, le chitosane, la carboxyméthylcellulose sodique et un carbomère ou des mélanges de ceux-ci,
**caractérisée en ce que** l'agent de viscosité est présent en une quantité de 0,02 % à 0,12 % m/v.

2. Composition liquide pour une utilisation selon la revendication 1, dans laquelle l'agent de viscosité est présent en une quantité de 0,06 % m/v.

3. Composition liquide pour une utilisation selon la revendication 1, dans laquelle l'alendronate monosodique trihydraté est présent en une quantité de 0,09 % à 0,27 % m/v.

4. Composition liquide pour une utilisation selon la revendication 1, dans laquelle l'agent aromatisant est susceptible de donner à la composition un goût de fruit.

5. Composition liquide pour une utilisation selon la revendication 4, dans laquelle l'agent aromatisant est un liquide aromatisant d'orange, un liquide de citron, un liquide de framboise, un liquide de pamplemousse ou un liquide de fruit rouge.

6. Composition liquide pour une utilisation selon la revendication 1, comprenant en outre un agent colorant.

7. Composition liquide pour une utilisation selon la revendication 6, dans laquelle l'agent colorant est un agent colorant jaune.

8. Composition liquide pour une utilisation selon la revendication 7, dans laquelle l'agent colorant est le jaune de quinoléine.

9. Composition liquide pour une utilisation selon la revendication 6, dans laquelle l'agent colorant est le rouge allura ou l'érythrosine.

10. Composition liquide pour une utilisation selon la revendication 1, dans laquelle l'agent aromatisant est présent en une quantité de 0,10 % à 0,50 % m/v, de préférence de 0,30 % à 0,50 % m/v.

11. Composition liquide pour une utilisation selon la revendication 6, dans laquelle l'agent colorant est présent en une quantité inférieure à 0,03 % m/v, de préférence comprise dans la plage allant de 0,005 % à 0,022 % m/v.

12. Composition liquide pour une utilisation selon la revendication 1, comprenant en outre un ou plusieurs agents édulcorants, un ou plusieurs agents conservateurs et/ou un ou plusieurs co-solvants.

13. Composition liquide pour une utilisation selon la revendication 12, dans laquelle l'agent édulcorant est choisi dans le groupe constitué par le cyclamate ou ses sels pharmaceutiquement acceptables, la saccharine ou ses sels pharmaceutiquement acceptables, le sucralose, l'aspartame, le dextrose, le maltose, le fructose, le galactose ou des mélanges de ceux-ci.

14. Composition liquide pour une utilisation selon la revendication 13, dans laquelle l'agent édulcorant est le cyclamate de sodium présent en une quantité de 0,10 % à 0,20 % m/v, de préférence 0,14 % m/v.

15. Composition liquide pour une utilisation selon la revendication 13, dans laquelle l'agent édulcorant est la saccharine de sodium présente en une quantité de 0,075 % à 0,60 % m/v, de préférence de 0,10 % à 0,20 % m/v.

16. Composition liquide pour une utilisation selon la revendication 13, dans laquelle l'agent édulcorant est le sucralose présent en une quantité de 0,010 % à 0,020 % m/v, de préférence de 0,015 % à 0,02 % m/v.

17. Composition liquide pour une utilisation selon la revendication 12, dans laquelle l'agent conservateur est choisi dans le groupe constitué par l'éthylparabène, le méthylparabène, le propylparabène, le méthylparabène de sodium, le propylparabène de sodium et le butylparabène de sodium.

18. Composition liquide pour une utilisation selon la revendication 17, dans laquelle le méthylparabène est présent en une quantité de 0,03 % à 0,1 % m/v et/ou le propylparabène est présent en une quantité de 0,01 % à 0,2 % m/v.

19. Composition liquide pour une utilisation selon la revendication 1, dans laquelle la composition est une suspension liquide.

20. Composition liquide pour une utilisation selon la revendication 1, dans laquelle le co-solvant est présent en une quantité de 1 % m/v, et est de préférence le propylène glycol.

21. Composition liquide pour une utilisation selon la revendication 1, ayant un pH compris dans la plage allant de 4,00 à 7,00.

22. Composition liquide pour une utilisation selon la revendication 1, ayant une densité de 0,980 à 1,02 g/ml à une température de 20 °C.

23. Utilisation d'une composition selon l'une quelconque des revendications précédentes, pour la préparation d'un médicament pour la prévention et/ou le traitement de maladies métaboliques osseuses.

24. Utilisation selon la revendication 23, pour une administration par impulsions.

25. Utilisation selon la revendication 23 ou 24, pour la prévention et/ou le traitement d'ostéopathies métaboliques ou de l'ostéoporose.

26. Utilisation selon l'une quelconque des revendications 23 à 25 chez les enfants.

27. Utilisation selon l'une quelconque des revendications 23 à 26 en tant que boisson avant l'absorption d'aliments.

28. Procédé de préparation d'une composition liquide selon l'une quelconque des revendications 1 à 22, comprenant les étapes consistant à :
(i) charger de l'eau purifiée dans un réacteur, ajouter l'agent conservateur et agiter jusqu'à complète dissolution, puis ajuster facultativement la température à 30 à 35 °C,
(ii) ajouter l'alendronate ou son sel pharmaceutiquement acceptable et agiter jusqu'à complète dissolution,
(iii) ajouter l'agent de viscosité et agiter jusqu'à complète dispersion du produit,
(iv) ajouter l'agent édulcorant et agiter jusqu'à complète dissolution,
(v) dissoudre l'agent colorant dans de l'eau purifiée dans un récipient séparé et ajouter le mélange obtenu dans l'étape (iv) jusqu'à l'obtention d'une homogénéisation du mélange,
(vi) ajouter l'agent aromatisant et agiter jusqu'à l'obtention d'une homogénéisation, la température après l'ajout étant inférieure à 30 °C,
(vii) ajouter de l'eau purifiée jusqu'à l'obtention d'un volume théorique du lot et agiter jusqu'à homogénéisation, et
(viii) verser dans les récipients appropriés jusqu'au volume théorique.
